# EUROPÄISCHE PATENTSCHRIFT

(11) **EP 0 331 871 B1**
(45) Veröffentlichungstag und Bekanntmachung des Hinweises auf die Patenterteilung: **01.12.1993**
(21) Anmeldenummer: 89100403.8
(22) Anmeldetag: 11.01.1989
(51) Int. Cl.: A61K 31/55

(54) **Antipsychotisch wirksames Imidazobenzodiazepin**
Imidazobenzodiazepine with antipsychotic activity
Imidazobenzodiazépine à activité antipsychotique

(30) Priorität: 15.01.1988 CH 143/88
(43) Veröffentlichungstag der Anmeldung: 13.09.1989
(73) Patentinhaber: F. HOFFMANN-LA ROCHE AG, 4002 Basel (CH)
(72) Erfinder: Merz, Walter, Dr., CH-4058 Basel (CH)
(74) Vertreter: Lederer, Franz, Dr.

(56) Entgegenhaltungen:
- EP-A- 0 059 391
- EP-A- 0 139 460
- EUROPEAN JOURNAL OF PHARMACOLOGY, Band 147, Nr. 2, März 1988, Seiten 283-285, Elsevier Science Publishers B.V. (Biomedical Division), Amsterdam, NL; J.R.M. HAIGH et al.: "RO 16-6028, a benzodiazepine receptor partial agonist, does not exhibit anticonvulsant tolerance in mice"
- ACTA PSYCHIATR. SCAND., Band 76, Nr. 6, 1987, Seiten 702-706; A.C. ALTAMURA et al.: "Clonazepam/haloperidol combination therapy in schizophrenia: A double blind study"
- NEUROPSYCHOBIOLOGY, Band 18, Nr. 1, 1987, Seiten 9-12, S. Karger AG, Basel, CH; S. COHEN et al.: "Adjunctive Benzodiazepines in acute schizophrenia"
- PHYSIOLOGY & BEHAVIOR, Band 41, Nr. 3, 1987, Seiten 247-255, Pergamon Journals Ltd; S.J. COOPER et al.: "Partial agonists acting at benzodiazepine receptors can be differentiated in tests of ingestional behaviour"
- EUROPEAN JOURNAL OF PHARMACOLOGY, Band 142, Nr. 3, Oktober 1987, Seiten 343-354, Elsevier Science Publishers B.V. (Biomedical Division), Amsterdam, NL; D.P. O'BRIEN et al.: "Inhibition of non-dopamine cells in the ventral tegmental area by benzodiazepines: relationship to A10 dopamine cell activity"
- PSYCHOPHARMACOLOGY, Band 93, Nr. 3, 1987, Seiten 365-368, Springer-Verlag; D.J. SANGER: "Further investigation of the stimulus properties of chlordiazepoxide and zolpidem. Agonism and antagonism by two novel benzodiazepines"
- J. HETEROCYCLIC CHEM., Band 24, November-Dezember 1987, Seiten 1599-1604; E.J. GLAMKOWSKI et al.: "Tetracyclic benzodiazepines. 4. Synthesis of the novel benzo[c]pyrrolo[1,2,3-ef][1,5]benzodiazepine ring system, and derivatives with potential antipsychotic activity"

## Beschreibung

Die vorliegende Erfindung betrifft die Verwendung von t-Butyl (S)-8-brom-11,12,13,13a-tetrahydro-9-oxo-9H-imidazo [1,5-a]pyrrolo[2,1-c][1,4]benzodiazepin-1-carboxylat der Formel 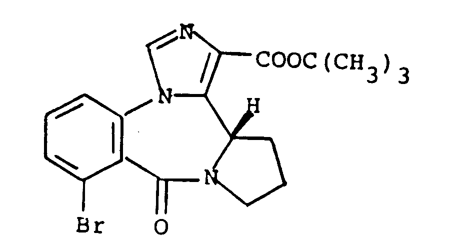
welche hiernach als Verbindung A bezeichnet wird, bei der Behandlung von psychotischen Erkrankungen des Menschen, insbesondere der Schizophrenie, und der Verhütung von Exazerbationen derselben. Die Verbindung A kann dabei als alleiniges Therapeutikum oder in Verbindung mit Neuroleptika, wie Haloperidol, eingesetzt werden.

Gegenstand der vorliegenden Erfindung sind: Die Verwendung der Verbindung A, gegebenenfalls in Verbindung mit Neuroleptika, bei der Behandlung von psychotischen Erkrankungen und der Verhütung von Exazerbationen derselben, die Verwendung der Verbindung A zur Herstellung von Mitteln zur Behandlung von psychotischen Erkrankungen und zur Verhütung von Exazerbationen derselben, sowie ein Verfahren und Mittel zur Behandlung von psychotischen Erkrankungen und zur Verhütung von Exazerbationen derselben.

Die Verbindung A ist eine bekannte Substanz, ihre Herstellung und ihre bekannten antikonvulsiven und anxiolytischen Eigenschaften werden beispielsweise in der Europäischen Patentpublikation Nr. 59 391 beschrieben.

Die folgende Beschreibung der Schizophrenie folgt den diagnostischen Kriterien der dritten, revidierten Ausgabe des amerikanischen statistischen und diagnostischen Handbuchs (Diagnostic and Statistical Manual of Mental Disorders, DSM-III-R) der amerikanischen psychiatrischen Gesellschaft (APA). Die Schizophrenie ist eine Geisteskrankheit unbekannter Genese, welche gewöhnlich im frühen Erwachsenenalter erstmals auftritt und sich auszeichnet durch eine Anzahl charakteristischer, psychotischer Symptome, eine familiäre Häufung, einen phasenhaften Verlauf und eine Verschlechterung im sozialen Verhalten und in den beruflichen Fähigkeiten in den Bereich unterhalb des höchsten je erreichten Niveaus. Als psychotische Symptome bezeichnet man Störungen des Denkinhalts (multiple, fragmentarische, inkohärente, unplausible oder schlechthin wahnhafte Inhalte oder Verfolgungsideen) und der Denkweise (Assoziationsverlust, Gedankenflucht, Inkohärenz bis hin zur Unverständlichkeit), ferner Störungen der Wahrnehmung (Halluzinationen), des Affekts (flache oder unangemessene Affekte) der Selbstempfindung, des Willens und Antriebs, der zwischenmenschlichen Beziehungen, und schliesslich Störungen der Psychomotorik (z.B. Katatonie). Eine Vielzahl anderer Symptome kann sich anschliessen.

Es wird zwischen prodromalen, aktiven und residualen Phasen unterschieden. Nach Zahl und Typ der Phasen unterscheidet man zwischen subchronischer Schizophrenie, subchronischer Schizophrenie mit akuter Exazerbation, chronischer Schizophrenie und chronischer Schizophrenie mit akuter Exazerbation. Nach der vorherrschenden psychotischen Symptomatik kann man schliesslich einen katatonen, einen desorganisiert-hebephrenen und einen paranoiden Typus der Schizophrenie sowie Mischformen der verschiedenen Typen unterscheiden.

Die vorherrschende, symptomatische Therapie der Schizophrenie sowie ähnlicher psychotischer Krankheiten, wie Paranoia, Schizo-affektive Psychosen, Schizophreniforme Psychose und andere Psychosen, ist die Pharmakotherapie mit Neuroleptika. Nachteile der Neuroleptika sind eine Vielzahl von - zum Teil irreversiblen - Nebenwirkungen, wie Parkinsonismus, Spätdyskinesie, Prolaktinerhöhung und ihre Folgen und anticholinerge Nebenwirkungen. Sie haben zudem eine in der Regel ungenügende oder ungünstige Wirkung auf Störungen des Affektes, des Antriebs, des Willens und der Psychomotorik ("negative" Symptome der Schizophrenie, im Gegensatz zu den sogenannten "produktiven" Symptomen, wie z.B. Halluzinationen oder Wahnideen).

Hochdosierte Benzodiazepine sind hin und wieder auf ihre antipsychotischen Wirkungen bei der Schizophrenie untersucht worden. Wo sich die Wirkung nicht von vornherein auf die angestammte anxiolytische Wirkung beschränkte, musste die hochdosierte Benzodiazepintherapie nach kurzer Zeit abgesetzt werden, weil entweder untolerierbare Nebenwirkungen auftraten oder die angewandte Therapie sich ohnehin im experimentellen, nicht ordnungsgemäss zugelassenen Dosierungsbereich bewegte.

Acta Psychiatr. Scand. 76, 702-706 (1987) beschreibt einen Vergleich einer Kombinationstherapie vom Clonazepam und Haloperidol mit einer reinen Haloperidoltherapie bei der Behandlung der Schizophrenie.

Neuropsychology 18, 9-12 (1987) berichtet einen klinischen Test betreffend die Wirkung von Lorazepam und/oder Triazolam in Verbindung mit einem Neuroleptikum bei der Behandlung von chronischer Schizophrenie.

Gemäss European J. Pharmacol. 142, 343-354 (1987) könnten möglicherweise Benzodiazepine als Zusatz zu Neuroleptika bei der Behandlung von Schizophrenie nützlich sein.

Ueberraschenderweise hat es sich gezeigt, dass die Verbindung A neben der bekannten antikonvulsiven und anxiolytischen eine ausgeprägt antipsychotische Wirksamkeit aufweist und zwar sowohl gegen die produktiven wie gegen die negativen Symptome. Sie zeichnet sich ferner durch sehr gute Verträglichkeit, insbesondere durch das Fehlen extrapyramidaler motorischer, anticholinerger und prolaktin-induzierter Nebenwirkungen aus.

Als Beispiel für die antipsychotische Wirkung der Verbindung A soll die Wirkung auf die Schizophrenie anhand einer einzelblinden Studie an 10 Patienten mit chronischer paranoider Schizophrenie demonstriert werden.

In dieser Studie wurde die Verbindung A in Form von Tabletten zu 0,5 mg Wirkstoffgehalt verwendet.

Die Patienten, welche nach den Kriterien des DSM-III ausgewählt worden waren, sollten während 4 Wochen eine verteilte Dosis von mindestens 1,5 mg täglich erhalten. Während der ersten Behandlungswoche konnte diese Tagesdosis nach Massgabe der Wirksamkeit und der Nebenwirkungen auf bis zu 4,5 mg hinauftitriert werden. Die optimale Dosis wurde während der Wochen 2 und 3 beibehalten und während der vierten Behandlungswoche schrittweise bis auf Null abgebaut. Die Behandlung wurde durch eine fünfte Woche abgeschlossen, während derer Placebo verabreicht wurde. Untersuchungstage waren die Tage 0, 1, 3, 7, 14, 21, 28, 30 und 35. An all diesen Behandlungstagen wurden die Daten nach der kurzen psychiatrischen Beurteilungsskala (Brief Psychiatric Rating Scale, BPRS; ein Verzeichnis psychotischer Symptome), der Angstskala nach Covi und der Depressionsskala nach Raskin, die Globalen Klinischen Impressionen (Clinical Global Impressions, CGI), die Vitalfunktionen und ein Verzeichnis der Nebenwirkungen erhoben. Haematologische und klinisch-chemische Parameter wurden vor Beginn und nach Abschluss der Behandlung mit der Verbindung A erhoben, gemeinsam mit einem EKG und einer medizinischen Allgemeinuntersuchung. Die Serumspiegel von Prolaktin wurden an den Tagen 0, 7, 14 und 21 gemessen, und die extrapyramidalen Nebenwirkungen wurden anhand der Skala von Simpson und Angus erhoben.

9 Männer und eine Frau mit einer akuten Exazerbation einer chronischen Schizophrenie wurden in die Studie eingeschlossen. Ihr mittleres Alter betrug 33 ± 4,9 Jahre, das mittlere Körpergewicht 68 ± 7,3 kg und die mittlere Körpergrösse 172 ± 4,8 cm. Zur Zeit des Einbezugs in die Studie hatte die Krankheit im Mittel schon 79 ± 66 Monate gedauert, und die Patienten waren im Durchschnitt 5,7 ± 3,3 mal deswegen hospitalisiert worden. Das Krankheitsgeschehen wurde bei den meisten Patienten als "schwer" beurteilt, eine Tatsache, welche auch durch den Anfangsskore von 65 Punkten in der BPRS unterstrichen wird.

Alle ausser einem Patienten reagierten sehr gut auf die Behandlung mit der Verbindung A, und zwar besser als auf die vorangegangene Behandlung mit Neuroleptika (in den meisten Fällen Haloperidol in anscheinend adäquater Dosierung). Die Zahl und die Intensität sowohl der produktiven wie der negativen psychotischen Symptome nahm vom dritten Tag der Behandlung an deutlich ab. Diese Verbesserung des klinischen Zustandes lässt sich gut an der BPRS ablesen: Vor Beginn der Behandlung betrug der mittlere Gesamtscore 65,5 Punkte, nach einem Tag Behandlung 60,8, nach 3 Tagen 37,4, nach 7 Tagen 13,3, nach 14 Tagen 9,5, nach 21 Tagen 7,1, und nach 28 Tagen Behandlung, am Ende der Ausschleichphase, noch 5,8 Punkte. Unter Placebo in der 5. Behandlungswoche war wieder ein leichter Anstieg zu verzeichnen (Tag 30: 7,4 Punkte; Tag 35: 11,2 Punkte). Das gleiche Bild ergibt sich bei den CGI: Schwere der Krankheit (Maximum 6, Minimum 0 Punkte): Vor Behandlung 4,9 Punkte, Tag 1: 4,6; Tag 3: 3,3; Tag 7: 1,8; Tag 14: 1,4; Tag 21: 1,2; Tag 28: 1,1; mit Wiederanstieg auf 1,2 am Tag 35; Therapeutische Effekte (Maximum 3, Minimum 0 Punkte): Tag 1: 0,6 Punkte; Tag 3: 2,2; Tag 7: 2,7; Tag 14: 2,8; Tage 21 und 28: 2,9 Punkte. Eine Verbesserung trat in allen Symptomen und Faktoren der BPRS ein, nicht nur in Symptomen der Angst und Depression. Die Verbesserung des funktionellen und sozialen Verhaltens der Patienten wurde nicht nur durch die psychiatrischen Skalen gezeigt, sondern auch durch die Verwandten der Patienten und die Patienten selbst bezeugt. Die Verbesserung dauerte über das Ende der Behandlung mit der Verbindung A an, da die Werte nach 7 Tagen Placebo-Behandlung (Tag 35) nur unwesentlich höher lagen als 7 Tage zuvor.

Die sehr gute Gesamtverträglichkeit der Verbindung A lässt sich anhand der CGI betreffend Nebenwirkungen ablesen (schlechtester Wert 3, bester Wert 0 Punkte): Nach einem Tag Behandlung betrug der Wert 0,1 Punkte; nach 3 Tagen 0,1, nach 7 Tagen 0,0; nach 14 Tagen 0,2 und nach 21 und 28 Tagen je 0,0 Punkte. Die Skala nach Simpson und Angus (Spannweite 0-9 Punkte) zeigt lediglich das Verschwinden der durch die vorangegangene Neuroleptika-Behandlung induzierten extrapyramidalen Störungen an: 1,1 Punkte am Tag 1; 1,1 am Tag 3; 0,6 an den Tagen 7 und 14 und 0,4 Punkte am Tag 28. Ein gleiches Verhalten zeigt der Serumprolaktin-Spiegel (Normalwerte <20 ng/ml): 21,5 ± 19.8 am Tag 0; 10,2 ± 8,3 am Tag 7; 5,7 ± 2,2 am Tag 14 und 6,7 ± 2,7 ng/ml am Tag 21.

Die Ergebnisse dieser Studie belegen eine antipsychotische Wirksamkeit der Verbindung A bei der paranoiden Schizophrenie, bei gleichzeitiger hervorragender Verträglichkeit. Es konnten ferner keine extrapyramidalen und prolaktinbedingten Nebenwirkungen beobachtet werden.

Im Rahmen der vorliegenden Erfindung wird die Verbindung A vorzugsweise in Form von peroral verabreichbaren pharmazeutischen Präparaten verwendet, z.B. in Form von Tabletten, Lacktabletten, Dragées, Hart- und Weichgelatinekapseln, Lösungen, Emulsionen oder Suspensionen. Als Dosierungsform werden Tabletten bevorzugt.

Zur Herstellung von pharmazeutischen Präparaten wird die Verbindung A mit pharmazeutisch inerten, anorganischen oder organischen Trägern verarbeitet. Als solche Träger kann man für Tabletten, Lacktabletten, Dragées und Hartgelatinekapseln beispielsweise Milchzucker, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Zur Herstellung von Lösungen und Sirupen eignen sich als Träger beispielsweise Wasser, Polyole, Saccharose, Invertzucker, Glukose und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten, beispielsweise die bereits erwähnten Neuroleptika.

Wie eingangs erwähnt, kann die Verbindung A bei der Behandlung von psychotischen Erkrankungen, insbesondere der Schizophrenie, und der Verhütung von Exazerbationen derselben verwendet werden. Die Dosierung kann je nach Schwere der Erkrankung, Alter und Gewicht des Patienten variieren und ist natürlich in jedem einzelnen Fall den individuellen Gegebenheiten anzupassen. Im allgemeinen dürfte bei oraler Verabreichung eine Tagesdosis von etwa 0,5 mg bis 6 mg angemessen sein.

Das nachfolgende Beispiel beschreibt eine für die praktische Anwendung der vorliegenden Erfindung geeignete Dosierungsform. Sie soll ihren Umfang jedoch in keiner Weise beschränken.

### Beispiel

| | |
|---|---|
| Verbindung A | 0,5 mg |
| Milchzucker | 126,5 mg |
| Maisstärke | 54,0 mg |
| Povidone K30 | 8,0 mg |
| Na-Carboxymethylstärke | 10,0 mg |
| Magnesiumstearat | 1,0 mg |
| | 2̅0̅0̅,̅0̅ m̅g̅ |

Die Verbindung A, der Milchzucker und die Maisstärke werden gemischt und mit einer wässrigen und/oder alkoholischen Lösung von Povidone granuliert. Das getrocknete und gebrochene Granulat wird mit Na-Carboxymethylstärke und Magnesiumstearat gemischt und anschliessend zu Tabletten von 200 mg gepresst.

## Patentansprüche

1. Verwendung der Verbindung A, t-Butyl (S)-8-brom-11,12,13,13a-tetrahydro-9-oxo-9H-imidazo [1,5-a]pyrrolo[2,1-c][1,4]benzodiazepin-1-carboxylat, der Formel 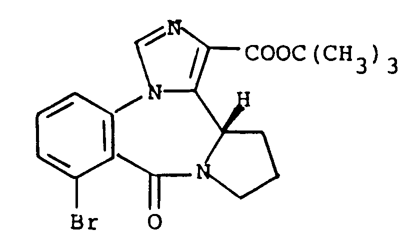 zur Herstellung von Mitteln zur Behandlung von psychotischen Erkrankungen, insbesondere der Schizophrenie, und zur Verhütung von Exazerbationen derselben.

## Claims

1. The use of compound A, t-butyl (S)-8-bromo-11,12,13,13a-tetrahydro-9-oxo-9H-imidazo[1,5-a]pyrrolo[2,1-c][1,4]benzodiazepine-1-carboxylate, of the formula 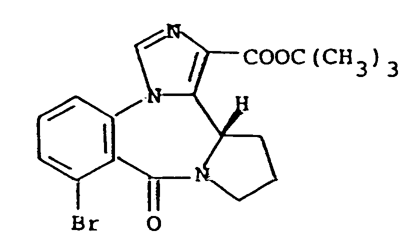 for the manufacture of medicaments for the treatment of psychotic disorders, especially of schizophrenia, and for the prevention of exacerbations thereof.

## Revendications

1. Application du composé A, le t-butyl (S)-8-bromo-11,12,13,13a-tétrahydro-9-oxo-9H-imidazo [1,5-a] - pyrrolo[2,1-c][1,4]benzodiazépine-1-carboxylate, de formule 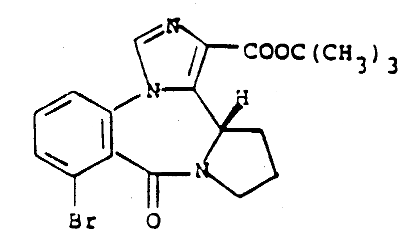 à la préparation d'agents pour le traitement des maladies psychotiques, en particulier de la schizophrénie, et pour la prévention de leurs exacerbations.
